Europäisches Patentamt

European Patent Office

Office européen des brevets

(11) Publication number: **0 322 989**
**A2**

# EUROPEAN PATENT APPLICATION

(21) Application number: 88203039.8

(22) Date of filing: 29.12.88

(51) Int. Cl.4: **C07C 31/04 , C07C 29/15**

(30) Priority: 30.12.87 GB 8730280 .

(43) Date of publication of application:
05.07.89 Bulletin 89/27

(84) Designated Contracting States:
BE DE ES FR GB IT NL

(71) Applicant: SHELL INTERNATIONALE
RESEARCH MAATSCHAPPIJ B.V.
Carel van Bylandtlaan 30
NL-2596 HR Den Haag(NL)

(72) Inventor: Sie, Swan Tiong
Badhuisweg 3
NL-1031 CM Amsterdam(NL)
Inventor: van Dijk, Arjan
Badhuisweg 3
NL-1031 CM Amsterdam(NL)

(74) Representative: Aalbers, Onno et al
P.O. Box 302
NL-2501 CH The Hague(NL)

(54) Process for the production of methanol.

(57) Process for the production of methanol by

(1) reacting CO with $H_2$ in the liquid phase in the presence of a nickel salt and an alcoholate of an alkali or alkaline earth metal, injecting an inert liquid coolant into said liquid phase, which coolant is sufficiently low boiling so as to allow substantially complete vaporization thereof upon contact with the liquid phase and which liquid is substantially immiscible with methanol,

(2) withdrawing a gaseous mixture of methanol and vaporized coolant from (1),

(3) condensing the gaseous mixture from (2), and

(4) separating the condensate from (3) into a liquid methanol phase and a liquid coolant phase.

EP 0 322 989 A2

# PROCESS FOR THE PRODUCTION OF METHANOL

The invention relates to a process for the production of methanol.

U.S. patent specifications Nos. 4,613,623, 4,614,749 and 4,619,946 concern processes for the production of methanol in the liquid phase by conversion of a gaseous mixture comprising carbon monoxide and hydrogen in the presence of a catalytic system obtainable by combining at least an alcoholate of an alkali metal or of an alkaline earth metal with a salt containing a cation of a metal of Group 8 of the Periodic Table of the Elements, for example nickel, palladium or cobalt. These known processes have the advantage that they can be carried out at a relatively low temperature and a relatively low pressure as compared with the currently used industrial scale methanol manufacturing processes which are operating at high temperatures and high pressures.

A problem to be solved in the known low-temperature processes described hereinbefore is the removal of heat developed by the conversion of carbon monoxide and hydrogen to methanol. It would, of course, be possible to remove this heat by means of indirect heat exchange with cooling water, thus producing hot water. This, however, has as a disadvantage that, a relatively small temperature difference being available, a large cooling surface would be required. Moreover, if a leak would occur, water would deactivate the catalytic system.

It is an object of the present invention to remove said heat in a simple manner and without the chance of deactivating the catalytic system.

Another object is to keep the liquid phase in which methanol is formed at a substantially uniform temperature.

A further object is to control the temperature of the liquid phase in a simple manner.

The invention, therefore, provides a process for the production of methanol which process comprises the following steps:-

step 1:- forming methanol in the liquid phase by conversion of a gaseous mixture comprising carbon monoxide and hydrogen in the presence of a catalytic system obtainable by combining at least

(a) a nickel salt, and

(b) an alcoholate of an alkali metal or of an alkaline earth metal,

injecting an inert liquid coolant into said liquid phase, which coolant is sufficiently low boiling so as to allow substantially complete vaporization thereof upon contact with the liquid phase and which liquid coolant is substantially immiscible with liquid methanol at a temperature in the range of from 0 °C to 70 °C,

step 2:- withdrawing a gaseous mixture comprising methanol and vaporized coolant from the liquid phase of step 1;

step 3:- cooling the gaseous mixture withdrawn in step 2 so as to form a condensate and, if required, further cooling the condensate to obtain a liquid methanol phase and a liquid coolant phase,

step 4:- separating the condensate formed in step 3 into the liquid methanol phase and the liquid coolant phase.

In the process according to the present invention the liquid phase in step 1 is brought into contact with a liquid coolant, simultaneously with the formation of methanol. The coolant evaporates from the liquid phase, together with methanol, and, as a result, both compounds withdraw heat of reaction from the liquid phase. Evaporation of methanol alone, not according to the invention, would not be sufficient for the removal of heat of reaction.

The method of removing the heat of reaction according to the present invention is simple, because it is not necessary to install cooling coils in the reactor in which step 1 is carried out or to provide for an external cooler through which the liquid phase would be circulated. None of such indirect methods of cooling is necessary, cooling taking place in a direct manner, by contacting the liquid phase with the liquid coolant. The liquid phase is brought into contact with the liquid coolant by simply injecting the coolant into the liquid phase. The agitation of the liquid phase caused by the liquid coolant evaporating very rapidly upon entering the liquid phase promotes obtaining a substantially uniform temperature throughout the liquid phase. The temperature of the liquid phase can be controlled by regulating the amount of liquid coolant with which it is brought into contact.

The coolant is inert which means that it does not interfere with the formation of methanol and it is substantially immiscible with methanol which means that it forms in step 3 a liquid methanol phase and a liquid coolant phase. The separation between those phases may be promoted by addition of relatively small amounts of a third component such as water. Examples of suitable coolants are n-pentane, 2-methylbutane, n-hexane, branched hexanes, perfluoropentane and perfluorohexane. Preference is given to n-pentane, because in view of its relatively low boiling point it easily evaporates in step 1. Therefore, according to a preferred embodiment of the present invention the coolant phase separated in step 4 is recycled to step 1, the methanol present in the recycled coolant phase doing no harm to the formation of methanol in step 1. Any coolant dissolved in the methanol phase is separated from methanol in the usual distillation step for purification of the methanol product and this amount of coolant may also be recycled to step 1. The temperature of the liquid phase in step 1 can simply be controlled by regulating the temperature and the amount of the recycled coolant phase.

Step 1 of the process according to the present invention is carried out at a relatively low temperature; preferably, the liquid phase in this step is kept at a temperature in the range of from 70 °C to 140 °C. Step 1 is preferably carried out at a pressure in the range of from 5 to 30 bar.

It will be appreciated that the catalytic system being used in step 1 may comprise, in addition to the components (a) and (b), one or more of the following components:-

(c) a hydride of an alkali metal or of an alkaline earth metal, and

(d) an alcohol or an in situ alcohol providing agent.

The in situ alcohol providing agent may be any compound which may form in situ an alcohol under the reaction conditions prevailing in step 1 and is preferably selected from alkyl formates, alkyl oxalates or alkyl carbonates or mixtures thereof. The alkyl groups preferably have in the range of from 1 to 5 carbon atoms. Preference is given to alkyl formates.

According to another embodiment of the process according to the present invention, the in situ formation of alcohol is achieved by addition of a predetermined amount of water, preferably a relatively small amount.

The alcohol to be used, if any, is preferably an aliphatic alcohol and, more preferably, an alkanol. Among the latter, alkanols having in the range of from 4 to 20 carbon atoms per molecule are preferred, for example tert-butyl alcohol, tert-pentyl alcohol, hexanol or heptanol. Dihydric alcohols, for example 1,4-butanediol, may be used, alone or in combination with alkanols.

The alcoholate to be used is preferably a sodium alcoholate or a potassium alcoholate. Among the alcoholates preference is given to alkanolates, particularly to those having in the range of from 1 to 20 carbon atoms per molecule. Particularly preferred are sodium methoxide, sodium ethoxide, sodium propoxide, sodium butoxide, sodium isobutoxide, potassium tert-butoxide, sodium tert-pentoxide and potassium 2-methyldodecanolate.

The anion of the salt in component (a) may be derived from a great variety of acids. It is preferred that the salt in component (a) is a salt of a carboxylic acid or a sulphonic acid. Among these acids preference is given to alkanoic acids having in the range of from 1 to 10 carbon atoms per molecule or to arylsulphonic acids, especially phenyl sulphonic acids. Particular preference is given to formic acid and acetic acid. Oxalic acid is another particularly preferred acid. Component (a) in step 1 is most preferably nickel formate, nickel acetate, nickel oxalate or nickel tosylate.

The salts in component (a) may contain crystal water.

The gaseous mixture being used in step 1 suitably contains carbon monoxide and hydrogen in a volume ratio in the range of from 1:10 to 10:1 and preferably 1:1.5 to 1:2.5, respectively. If desired, the gaseous mixture may contain an inert gas, for example nitrogen.

The gaseous mixture withdrawn in step 2 of the process according to the present invention contains carbon monoxide, hydrogen, methanol, methyl formate and coolant. This mixture is cooled in step 3 to such a temperature that a condensate is formed, the condensate comprising methanol, methyl formate and coolant. It is a feature of the present invention that cooling water having ambient temperature, for example 10-20 °C, can usually be applied for indirect cooling of the gaseous mixture. The carbon monoxide and hydrogen remaining after the cooling of step 3 may be given any desired destination but are preferably recycled to step 1 for formation of further quantities of methanol.

The condensate formed in step 3 may, if required after further cooling, be separated in step 4 in any desired manner, for example by gravity settling, which is preferred, or by centrifugation. The separation is simple in view of the relatively large difference in density between methanol and the examples of coolants given hereinbefore.

The process according to the present invention provides an economically attractive industrial bulk manufacturing process for the preparation of methanol, starting from cheap materials and operating at attractive economical and environmental conditions, i.e. using rather simple equipment and giving a significant reduction of the methanol cost price. The methanol produced may be used as fuel or as a starting material for further chemical synthesis.

The invention is further illustrated by the following example.

## Example

A catalyst system is prepared as follows. 100 parts by weight (pbw) of "diglyme" (2,5,8-trioxanonane), 4.1 pbw of nickel formate.$2H_2O$, 3.2 pbw of sodium hydride as a suspension in paraffin oil containing 80% NaH and 5.9 pbw of tert-pentyl alcohol are added together under an inert (N₂)-atmosphere. The mixture is heated to a temperature of 45 °C while stirring and kept at this temperature for 30 minutes before adding a solution consisting of 100 pbw of diglyme, 5.9 pbw of tert-pentyl alcohol and 21.7 pbw of methyl formate. The liquid mixture is then introduced into a methanol synthesis reactor where it is contacted with a gaseous mixture of 66.7 %v of $H_2$ and 33.3 %v of CO at a pressure of 15 bar and a temperature of 120 °C.

Synthesis feed gas of the above mentioned composition ($H_2$/CO = 2 v/v) is fed to the reactor at a rate of 750 $Nm^3$ per $m^3$ of liquid catalyst per hour. At the stated temperature and pressure, 56 %v of the synthesis feed gas is converted to methanol, at a methanol production rate of about 200 kg per $m^3$ catalyst volume per hour.

The methanol synthesis reactor consists of a pressure vessel provided with an inlet distribution system for feed gas as well as for liquid coolant. Fresh catalyst is added and a corresponding part of the catalytst inventory withdrawn during operation so as to control the volume of the catalyst inventory and its steady state activity. To keep the reaction temperature constant, a liquid coolant consisting substantially of n-pentane and having a temperature of 20 °C is injected into the reactor. The space velocity of the liquid coolant stream is 1000 $kg/m^3 \cdot h$.

The effluent gas from the reactor, which contains unconverted carbon monoxide, unconverted hydrogen, methanol vapour and n-pentane vapour is cooled, thereby yielding a condensate consisting of n-pentane and methanol which upon addition of 1 %w of water and further cooling to about 20 °C gives rise to two liquid phases which are separated from each other by gravity. The upper, pentane-rich phase consists of about 98 %w pentane with 2 %w of methanol, whereas the lower, methanol-rich, phase is composed of approximately 85 %w of methanol, 5 %w of n-pentane and 10 %w of water. Methanol from the latter phase is recovered in a pure form by distillation, which also recovers the n-pentane from the methanol-rich phase. This quantity of n-pentane which amounts to about 1% of the n-pentane in the pentane-rich phase mentioned hereinbefore, is added to the latter phase and the combined stream is returned to the reactor as coolant.

## Claims

1. A process for the production of methanol which process comprises the following steps:-

step 1:- forming methanol in the liquid phase by conversion of a gaseous mixture comprising carbon monoxide and hydrogen in the presence of a catalytic system obtainable by combining at least
(a) a nickel salt, and
(b) an alcoholate of an alkali metal or of an alkaline earth metal,
injecting an inert liquid coolant into said liquid phase, which coolant is sufficiently low boiling so as to allow substantially complete vaporization thereof upon contact with the liquid phase and which liquid coolant is substantially immiscible with liquid methanol at a temperature in the range of from 0 °C to 70 °C,

step 2:- withdrawing a gaseous mixture comprising methanol and vaporized coolant from the liquid phase of step 1;

step 3:- cooling the gaseous mixture withdrawn in step 2 so as to form a condensate and, if required, further cooling the condensate to obtain a liquid methanol phase and a liquid coolant phase,

step 4:- separating the condensate formed in step 3 into the liquid methanol phase and the liquid coolant phase.

2. A process as claimed in claim 1 in which as inert liquid coolant liquid n-pentane is used.

3. A process as claimed in claim 1 or 2 in which the liquid coolant phase separated in step 4 is used as inert liquid coolant in step 1.

4. A process as claimed in any one of the preceding claims in which the liquid phase in step 1 is kept at a temperature in the range of from 70 °C to 140 °C, and at a pressure in the range of from 5 to 30 bar.

5. A process as claimed in any one of the preceding claims in which the catalytic system is obtainable by combining in addition to the components (a) and (b) one or more of the components
(c) a hydride of an alkali metal or of an alkaline earth metal, and
(d) an alcohol or an in situ alcohol providing agent.

6. A process as claimed in claim 5 in which the in situ alcohol providing agent is selected from alkyl formates, alkyl oxalates or alkyl carbonates or a mixture thereof.

7. A process as claimed in claim 6 in which the in situ alcohol providing agent is an alkyl formate.

8. A process as claimed in claim 5 in which the in situ alcohol providing agent is a predetermined amount of water.

9. A process as claimed in any one of the preceding claims in which as component (a) in step 1 nickel formate, nickel acetate, nickel oxalate or nickel tosylate is used.

10. A process as claimed in any one of the preceding claims in which as component (b) in step 1 a sodium alcoholate or a potassium alcoholate is used, preferably sodium methoxide, sodium ethoxide, sodium propoxide, sodium butoxide, sodium isobutoxide, sodium tert-pentoxide, potassium tert-butoxide or potassium 2-methyl-dodecanolate.